# EUROPÄISCHE PATENTSCHRIFT

(11) **EP 1 085 007 B1**
(45) Veröffentlichungstag und Bekanntmachung des Hinweises auf die Patenterteilung: **12.06.2002**
(21) Anmeldenummer: 00116005.0
(22) Anmeldetag: 26.07.2000
(51) Int. Cl.: C07C 67/035, C07C 67/54

(54) **Verfahren zur Herstellung von Cyclooctanol**
Process for the preparation of cyclooctanol
Procédé de préparation du cyclooctanol

(30) Priorität: 18.09.1999 DE 19944874
(43) Veröffentlichungstag der Anmeldung: 21.03.2001
(73) Patentinhaber: Degussa AG, 40474 Düsseldorf (DE)
(72) Erfinder: Kaufhold, Manfred, Dr., 45770 Marl (DE); Köhler, Günther, Dr., 45770 Marl (DE); Paulczynski, Renate, 44623 Herne (DE); Beuth, Michael, 46282 Dorsten (DE)

(56) Entgegenhaltungen:
- EP-A- 0 829 466
- GB-A- 1 153 468

## Beschreibung

Die Erfindung betrifft ein Verfahren zur Herstellung von Cyclooctanol durch Umsetzung von Cycloocten mit Ameisensäure zu Cyclooctylformiat, Phasentrennung nach der Reaktion in eine untere an Ameisensäure reiche A-Phase (Ameisensäuregehalt > 60 %) und eine obere an Ameisensäure arme B-Phase (Ameisensäuregehalt < 20 %), Extraktion der A-Phase, Zusammenführen dieses Extraktes mit der B-Phase, schonende destillative Aufarbeitung des das Cyclooctylformiat enthaltenden Reaktionsgemisches über einen kurzen Weg sowie Umesterung des Cyclooctylformiats zu Cyclooctanol.

Cyclooctanol ist ein wichtiges Zwischenprodukt zur Herstellung von u. a. Cyclooctanon, das im Pharmabereich benötigt wird, und zur Herstellung von Riechstoffen. Synthesen von Cyclooctanol aus Cycloocten über das Formiat sind aus der Literatur bekannt. So beschreibt GB 1 153 468 ein Verfahren, bei dem Ameisensäure ohne Katalysator addiert, das Reaktionsgemisch destillativ aufgearbeitet und das so gewonnene Cyclooctylformiat mit Natronlauge verseift wird. Es wird darauf hingewiesen, daß die Temperaturen bei der Destillation sehr niedrig sein müssen. Um Zersetzungen zu vermeiden, werden Temperaturen von weniger als 100 °C gefordert. Diese Forderung ist aber bei einer technischen Durchführung nicht realisierbar und selbst im Beispiel 1 wird als Siedepunkt des Formiats eine über 100 °C liegende Temperatur genannt. Die relativ schlechte Ausbeute von nur 82 % bezogen auf umgesetztes Cycloocten ist auf thermische Zersetzung zurückzuführen. Ein weiterer Nachteil dieses Verfahrens ist, daß bei der Verseifung des Formiats mit Natronlauge mindestens stöchiometrische Mengen an Natriumformiat entstehen, die entsorgt werden müssen.

Aufgabe der Erfindung war es daher, ein Verfahren zu finden, das technisch leicht realisierbar ist, bei dem keine thermische Zersetzung auftritt und bei dem keine nennenswerten Mengen Salze als Abfall anfallen.

Die Aufgabe wird erfindungsgemäß dadurch gelöst, daß die Umsetzung von Cycloocten mit Ameisensäure in Abwesenheit eines zugesetzten Katalysators durchgeführt wird, die Destillation kontinuierlich ohne Kolonne über einen kurzen Weg erfolgt und der so erhaltene Ester mit einem Alkohol umgeestert wird.

Überraschend wurde insbesondere gefunden, daß die Destillation ohne großen Trennaufwand in einer Appatur mit einem kurzen Destillationsweg durchgeführt werden kann, ohne daß dabei thermische Zersetzung stattfindet und daß der so erhaltene Rohester nur wenig oder gar keine Ameisensäure enthält, so daß der Ester in an sich bekannter Weise mit einem Alkohol katalytisch zum Cyclooctanol umgeestert werden kann.

Ferner wurde gefunden, daß es wegen der Instabilität der Ameisensäure günstig ist, die Hauptmenge an nichtumgesetzter Ameisensäure vor der Destillation des Cyclooctylformiats abzutrennen und getrennt aufzuarbeiten. Das geschieht vorteilhafterweise dadurch, daß zunächst eine Phasentrennung in die Phasen A und B und eine Extraktion des Cyclooctylformiats aus der Phase A erfolgt. Der Extrakt wird zur Phase B gegeben und in die destillative Aufarbeitung des Cyclooctylformiats geführt. Die Ameisensäure wird in einer getrennten Kolonne destilliert und als Kopfprodukt reine Ameisensäure, die wieder in die Reaktion eingesetzt werden kann, erhalten. Als Sumpfprodukt verbleibt ein Wasser-Säure-Gemisch.

Gegenstand der Erfindung ist daher ein Verfahren zur Herstellung von Cyclooctanol aus Cycloocten mit folgenden Reaktions- und Verfahrensschritten:
a) Umsetzung von Cycloocten mit Ameisensäure zu Cyclooctylformiat in Abwesenheit eines zugesetzten Katalysators,
b) Trennung der beiden Phasen in eine Phase A und eine Phase B,
c) Extraktion von Cyclooctylformiat aus der Phase A mit einem unpolaren Lösemittel,
d) Zusammenführen der Phase B und des Extraktes aus Phase A,
e) destillative Aufarbeitung der Phase B und des Extraktes in einer Apparatur mit einem kurzen Destillationsweg und
f) katalytische Umesterung des Cyclooctylformiats mit einem Alkohol zum Cyclooctanol.

Das erfindungsgemäße Verfahren benötigt in der ersten Stufe weder Lösemittel noch extern zugesetzten Katalysator.

Das erfindungsgemäße Verfahren hat den Vorteil, daß eine Kolonne eingespart wird und trotzdem die bei der Reaktion in der Regel im großen Überschuß vorhandene Ameisensäure vom Ester vollständig abgetrennt wird. Erst dadurch wird es möglich, den Ester mit kleinen Mengen eines Katalysators umzuestern, ohne dabei nennenswerte Mengen an Salz als Abfall zu produzieren. Als Katalysatoren für die Umesterung können vorzugsweise alkalische Alkali- und Erdalkaliverbindungen wie z. B. Alkali- und Erdalkalialkoholate verwendet werden. Als Beispiele seien Natriummethylat, Natriumethylat und Kaliummethylat genannt.

Das Molverhältnis Cycloocten zu Ameisensäure beträgt 1 : 1 bis 1 : 6, vorzugsweise 1 : 2 bis 1 : 4.

Die Reaktionstemperatur liegt zwischen 60 und 100 °C, vorzugsweise zwischen 70 und 90 °C. Eine Reaktionstemperatur von etwa 80 °C wird besonders bevorzugt. Das Verfahren kann diskontinuierlich oder kontinuierlich durchgeführt werden.

Nach der Reaktion, die bei diskontinuierlicher Fahrweise in 2 bis 10 Stunden, vorzugsweise in 4 bis 8 Stunden, beendet ist, wird die Reaktionsmischung abgekühlt. Nach Trennung der Phasen wird die an Ameisensäure reiche Phase A mit einem unpolaren Lösemittel extrahiert. Als Lösemittel kommen aliphatische Kohlenwasserstoffe (linear oder verzweigt) mit 5 bis 10 Kohlenstoffatomen wie z. B. Pentan, Hexan und Octan, cycloaliphatische Kohlenwasserstoffe wie z. B. Cyclohexan, aromatische Kohlenwasserstoffe wie z. B. Benzol oder Toluol und (cyclo)olefinische Kohlenwasserstoffe wie z. B. Cycloocten in Frage. Cycloocten wird bevorzugt eingesetzt, weil es bereits Bestandteil des Reaktionssystems ist.

Die destillative Aufarbeitung erfolgt in einem Verdampfer mit kurzem Destillationsweg wie z. B. einem Fallfilmverdampfer, einem Kurzwegverdampfer oder einem Dünnschichtverdampfer.

Nach der Extraktion wird die rohe Ameisensäure in einer Kolonne mit mindestens 10 theoretischen Böden aufgearbeitet, wobei eine nahezu wasserfreie Säure abdestilliert und im Sumpf ein höhersiedendes Ameisensäure-Wasser-Gemisch verbleibt, das entsorgt wird, was z. B. durch Verbrennen geschehen kann. Das Wasser entsteht dabei durch geringe thermische Zersetzung der Ameisensäure während der Reaktion. Es muß abgetrennt werden, weil es bei der Umsetzung mit dem Cycloocten stört. Der Wassergehalt der für die Reaktion eingesetzten Ameisensäure sollte daher vorzugsweise < 1 Gew.-% sein.

Die folgenden Beispiele sollen das erfindungsgemäße Verfahren näher erläutern, nicht jedoch auf die speziell genannten Umstände einschränken.

### Beispiel 1

### Diskontinuierliche Fahrweise

Es wurde eine 50 l Glasrührblase mit Rührer, Thermometer und einer Beheizung mit einem Öl-Thermostaten verwendet. In der Blase wurden 22 kg (0,2 kmol) Cycloocten und 28 kg (0,6 kmol) Ameisensäure zusammengegeben und bei 80 °C 6 Stunden intensiv gerührt. Nach Abkühlung auf < 30 °C wurden die Phasen, die sich in wenigen Minuten auftrennten, separiert. Als obere B-Phase wurden 22,7 kg mit

| | |
|---|---|
| HCOOH | 7,6 % |
| COF | 26,3 % |
| COE | 60,4 % |
| H₂O | 0,1 % |
| Rest | 5,6 % |

und als untere A-Phase 27,3 kg mit

| | |
|---|---|
| HCOOH | 78,7% |
| COF | 16,8 % |
| COE | 3,4 % |
| H₂O | 0,7 % |
| Rest | 0,4 % |

erhalten
HCOOH = Ameisensäure, COF = Cyclooctylformiat, COE = Cycloocten,
H₂O = Wasser

Die untere Phase wurde dreimal mit je 8,5 kg COE in einer 25 1 Rührapparatur extrahiert. Die Phasen wurden separiert und analysiert:

Den Extraktionsverlauf zeigt folgende Tabelle:

| **Phase** | **Menge (kg)** | **H**_{**2**}**O (%)** | **HCOOH (%)** | **COE (%)** | **COF (%)** | **Rest (%)** |
|---|---|---|---|---|---|---|
| 1. obere | 12,5 | 0,09 | 4,94 | 73,39 | 18,42 | 3,16 |
| 2. obere | 10,1 | 0 | 2,40 | 85,45 | 9,32 | 2,83 |
| 3. obere | 9,4 | 0 | 1,46 | 91,18 | 4,32 | 3,04 |
| 1. untere | 23,3 | 0,83 | 88,63 | 1,73 | 8,67 | 0,14 |
| 2. untere | 21,7 | 0,92 | 93,50 | 1,24 | 4,20 | 0,14 |
| 3. untere | 20,8 | 0,97 | 95,56 | 1,04 | 2,06 | 0,37 |

Die Gesamtmenge an COF in allen oberen Phasen betrug 61,6 mol. Daraus errechnet sich eine Ausbeute von 30,8 %, bezogen auf eingesetztes COE: Die Selektivität betrug 99,9 %, d. h. es war keine thermische Zersetzung während der Reaktion und ersten Aufarbeitung zu beobachten.

### Beispiel 2

### 2.1 Reaktion, kontinuierliche Fahrweise

Es wurde eine 30 l Glasrührblase mit Rührer, Thermometer und zwei Zulaufvorlagen verwendet, zur Beheizung diente ein Öl-Thermostat. Die Reaktion wurde kontinuierlich durchgeführt und zunächst in der Blase 11,0 kg (100 mol) Cycloocten und 14,0 kg (304 mol) Ameisensäure (99,9 %ig) vorgelegt, auf 80 °C erwärmt und 6 Stunden bei 80 °C intensiv gerührt.

Für den kontinuierlichen Betrieb wurden
2,6 l/h = 2,2 kg/h (20 mol/h) Cycloocten und
2,3 l/h = 2,8 kg/h (60 mol/h) Ameisensäure
in die Blase gepumpt. Insgesamt wurden über einen Zeitraum von 18 Stunden 50,4 kg Ameisensäure und 39,6 kg Cycloocten kontinuierlich zudosiert und im gleichen Maße eine Reaktionsmischung abgezogen, die zur Phasentrennung in einen Absitzbehälter geleitet wurde.

Als Reaktionsaustrag wurden 43,0 kg obere Phase und 46,2 kg untere Phase mit folgenden Zusammensetzungen erhalten:

| | **COE (%)** | **COF (%)** | **HCOOH (%)** | **Rest (%)** |
|---|---|---|---|---|
| obere Phase | 64,7 | 21,1 | 7,0 | 7,2 |
| untere Phase | 2,2 | 10,9 | 84,9 | 2,0 |

Hieraus errechnet sich eine Ausbeute von 26,0 %, bezogen auf eingesetztes COE und von 99,9 %, bezogen auf umgesetztes COE.

### 2.2 Destillative Aufarbeitung der Ameisensäure

Verwendet wurde eine kontinuierliche Destillationskolonne mit mindestens 10 theoretischen Böden. Es wurde eine extrahierte Ameisensäurephase, 207,5 kg, mit folgender Zusammensetzung destilliert:

| | |
|---|---|
| HCOOH | 98,5 % |
| H₂O | 0,9 % |
| COF | 0,5 % |
| COE | 0,1 % |

Als Destillat wurde 178,6 kg Ameisensäure mit 0,003 % Wasser erhalten. Im Sumpf verblieben 22,8 kg Ameisensäure-Wasser-Gemisch mit 10,6 % Wasser. COF wird bei der Destillation zu COE und HCOOH zersetzt. Das COE ist zu ca. 0,3 % im Destillat vorhanden. Es fand nur eine geringfügige Zersetzung der Ameisensäure statt.

### 2.3 Aufarbeitung von rohem Cyclooctylformiat mit Hilfe eines Dünnschichtverdampfers

Das nach der Extraktion vorliegende rohe Cyclooctylformiat enthielt noch 61,0 % Cycloocten und geringe Mengen an Ameisensäure, die unter schonenden Bedingungen in einem Dünnschichtverdampfer aus Glas mit 0,1 m² Oberfläche abdestilliert wurden. Die Destillationsbedingungen waren:

| | |
|---|---|
| Übergangstemperatur | 80 °C |
| Ölvorlauftemperatur | 145 °C |
| Druck | 100 hPa |
| Zulaufmenge | 3,0 l/h |

Einsatz, 75,3 kg, mit folgenden Komponenten:

| | |
|---|---|
| HCOOH | 7,3 % |
| COF | 22,8 % |
| COE | 61,0 % |
| Rest | 8,9 % |

Als Destillate wurden erhalten:
obere Phase, 51,7 kg und untere Phase 6,0 kg

Zusammensetzung:

| | **COF (%)** | **COE (%)** | **HCOOH (%)** | **Rest (%)** |
|---|---|---|---|---|
| obere Phase | 7,7 | 82,5 | 0,7 | 9,1 |
| untere Phase | 3,8 | 0,4 | 90,7 | 5,1 |

Das Sumpfprodukt, 17,5 kg, enthielt 78,6 % Cyclooctylformiat und 5,5 % Cycloocten, jedoch keine Ameisensäure. Es war keine Zersetzung zu beobachten.

### 2.4 Umesterung des Cyclooctylformiats zu Cyclooctanol

Da das aufkonzentrierte Cyclooctylformiat keine Ameisensäure enthielt, konnte es in üblicher Weise mit Methanol und Natriummethylat als Katalysator umgeestert werden.

Eingesetzt wurden 9,95 kg Sumpfprodukt mit o. g. Zusammensetzung, 6,41 kg Methanol und 3 mal 200 ml Natriummethylatlösung 30 %ig, die jeweils nach 2 Stunden zudosiert wurde. Bei Sumpftemperaturen um 53 °C bildete sich laufend Methylformiat, das abdestilliert wurde. Bereits nach 6 Stunden war die Reaktion beendet. Die destillative Aufarbeitung lieferte 5,28 kg Cyclooctanol mit einer Reinheit von 99,8 %. Ausbeute: 82,2 % bezogen auf eingesetztes Cyclooctylformiat. Die Gesamtausbeute über alle Fraktionen betrug 96,2 %.

## Patentansprüche

1. Verfahren zur Herstellung von Cyclooctanol aus Cycloocten umfassend die Reaktions- und Verfahrensschritte
a. Umsetzung von Cycloocten mit Ameisensäure zu Cyclooctylformiat in Abwesenheit eines zugesetzten Katalysators,
b. Trennung der beiden Phasen in eine Phase A und eine Phase B,
c. Extraktion von Cyclooctylformiat aus der Phase A,
d. Zusammenführen der Phase B und des Extraktes aus Phase A,
e. destillative Aufarbeitung der Phase B und des Extraktes in einer Apparatur mit einem kurzen Destillationsweg und
f. katalytische Umesterung des Cyclooctylformiats mit einem Alkohol zum Cyclooctanol.

2. Verfahren nach Anspruch 1,
**dadurch gekennzeichnet,**
**daß** das Molverhältnis Cycloocten zu Ameisensäure 1: 2 bis 1 : 4 beträgt.

3. Verfahren nach Anspruch 1 oder 2,
**dadurch gekennzeichnet,**
**daß** der Wassergehalt der eingesetzten Ameisensäure < 1 Gew.-% ist.

4. Verfahren nach Anspruch 1,
**dadurch gekennzeichnet,**
**daß** die Reaktionstemperatur für die Umsetzung von Cycloocten und Ameisensäure zwischen 60 °C und 100 °C liegt.

5. Verfahren nach Anspruch 1,
**dadurch gekennzeichnet,**
**daß** entstandenes in der Ameisensäure gelöstes Wasser durch Destillation weitgehend entfernt und die nahezu wasserfreie Ameisensäure in die Reaktion zurückgeführt wird.

6. Verfahren nach mindestens einem der vorhergehenden Ansprüche,
**dadurch gekennzeichnet,**
**daß** man als Lösemittel aliphatische, cycloaliphatische oder aromatische Kohlenwasserstoffe zum Extrahieren verwendet.

7. Verfahren nach mindestens einem der vorhergehenden Ansprüche,
**dadurch gekennzeichnet,**
**daß** man als Lösemittel Cycloocten zum Extrahieren verwendet.

8. Verfahren nach mindestens einem der vorhergehenden Ansprüche,
**dadurch gekennzeichnet,**
**daß** man für die Aufarbeitung des Cyclooctylformiats als Destillationsapparatur einen Dünnschichtverdampfer einsetzt.

9. Verfahren nach mindestens einem der vorhergehenden Ansprüche,
**dadurch gekennzeichnet,**
**daß** man für die Aufarbeitung des Cyclooctylformiats als Destillationsapparatur einen Fallfilmverdampfer benutzt.

10. Verfahren nach mindestens einem der vorhergehenden Ansprüche,
**dadurch gekennzeichnet,**
**daß** man für die Aufarbeitung des Cyclooctylformiats als Destillationsapparatur einen Kurzwegverfampfer benutzt.

## Claims

1. A process for the preparation of cyclooctanol from cyclooctene comprising the reaction and process steps
a. reaction of cyclooctene with formic acid to give cyclooctyl formate in the absence of an added catalyst,
b. separation of the two phases into phase A and phase B,
c. extraction of cyclooctyl formate from phase A,
d. combination of phase B and the extract from phase A,
e. distillative work-up of phase B and of the extract in an apparatus fitted with a short distillation path and
f. catalytic transesterification of the cyclooctyl formate with an alcohol to give cyclooctanol.

2. A process according to claim 1,
**characterized in that**
the molar ratio of cyclooctene to formic acid is from 1:2 to 1:4.

3. A process according to claim 1 or 2,
**characterized in that**
the water content of the formic acid used is < 1% by weight.

4. A process according to claim 1,
**characterized in that**
the reaction temperature for the reaction of cyclooctene and formic acid is between 60°C and 100°C.

5. A process according to claim 1,
**characterized in that**
water which has formed and dissolved in the formic acid is largely removed by distillation, and the virtually anhydrous formic acid is returned to the reaction.

6. A process according to at least one of the preceding claims,
**characterized in that**
the solvent used for the extraction is an aliphatic, cycloaliphatic or aromatic hydrocarbon.

7. A process according to at least one of the preceding claims,
**characterized in that**
the solvent used for the extraction is cyclooctene.

8. A process according to at least one of the preceding claims,
**characterized in that**
a thin-film evaporator is employed as distillation apparatus for work-up of the cyclooctyl formate.

9. A process according to at least one of the preceding claims,
**characterized in that**
a falling-film evaporator is used as distillation apparatus for work-up of the cyclooctyl formate.

10. A process according to at least one of the preceding claims,
**characterized in that**
a short-path evaporator is used as distillation apparatus for work-up of the cyclooctyl formate.

## Revendications

1. Procédé de préparation de cyclooctanol à partir de cyclooctène comprenant les étapes de réaction et de procédé :
a) réaction du cyclooctène avec l'acide formique en formiate de cyclooctyle en l'absence d'un catalyseur ajouté,
b) séparation des deux phases en une phase A et une phase B,
c) extraction du formiate de cyclooctyle de la phase A,
d) de la phase B et de l'extraction provenant de la phase A,
e) traitement par distillation de la phase B et de l'extraction, dans un appareillage ayant une distillation flash, et
f) transesterification catalytique du formiate de cyclooctyle avec un alcool en cyclooctanol.

2. Procédé selon la revendication 1,
**caractérisé en ce que**
le rapport molaire du cyclooctène à l'acide formique s'élève à 1 : 2 à 1 : 4.

3. Procédé selon la revendication 1 ou la revendication 2,
**caractérisé en ce que**
la teneur en eau de l'acide formique mis en oeuvre est < 1 % en poids.

4. Procédé selon la revendication 1,
**caractérisé en ce que**
la température de réaction pour la réaction du cycloocténe et de l'acide formique se situe entre 60°C et 100°C.

5. Procédé selon la revendication 1,
**caractérisé en ce que**
l'eau dissoute qui s'est formée dans l'acide formique est largement éliminée par distillation et on ramène l'acide formique à peu près anhydre dans la réaction.

6. Procédé selon au moins l'une des revendications précédentes,
**caractérisé en ce qu'**
on utilise comme solvants des hydrocarbures aliphatiques, cycloaliphatiques ou aromatiques en vue de l'extraction.

7. Procédé selon au moins l'une des revendications précédentes,
**caractérisé en ce qu'**
on utilise comme solvant le cyclooctène en vue de l'extraction.

8. Procédé selon au moins l'une des revendications précédentes,
**caractérisé en ce qu'**
on met en oeuvre pour le traitement du formiate de cyclooctyle, en tant qu'appareillage de distillation, un évaporateur en couche mince.

9. Procédé selon au moins l'une des revendications précédentes,
**caractérisé en ce qu'**
on utilise pour le traitement du formiate de cyclooctyle en tant qu'appareillage de distillation un évaporateur à film tombant.

10. Procédé selon au moins l'une des revendications précédentes,
**caractérisé en ce qu'**
on utilise pour le traitement du formiate de cyclooctyle en tant qu'appareillage de distillation un évaporateur flash.
